# FASCICULE DE BREVET EUROPEEN

(11) **EP 1 380 590 B1**
(45) Date de publication et mention de la délivrance du brevet: **06.09.2006**
(21) Numéro de dépôt: 03292131.4
(22) Date de dépôt: 29.08.2003
(51) Int. Cl.: C07K 5/06, C07K 5/02, C07D 209/42

(54) **Nouveau procédé de synthèse du perindopril et de ses sels pharmaceutiquement acceptables**
Verfahren für die Synthese von Perindopril und seiner pharmazeutisch annehmbaren Salzen ¬2003/26|
Method for synthesis of perindopril and its pharmaceutically acceptable salts

(43) Date de publication de la demande: 14.01.2004
(73) Titulaire: Les Laboratoires Servier, 92415 Courbevoie Cedex (FR)
(72) Inventeur: Dubuffet, Thierry, 76190 Autretot (FR); Lecouve, Jean-Pierre, 76600 Le Havre (FR)

(56) Documents cités:
- EP-A- 1 319 668
- EP-A- 1 321 471

## Description

La présente invention concerne un procédé de synthèse du perindopril de formule (I): 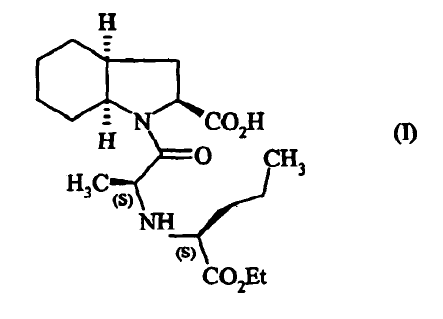 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de l'ester benzylique de l'acide (2S, 3aS, 7aS)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.

La demanderesse a présentement mis au point un nouveau procédé de synthèse du perindopril.

Plus spécifiquement, la présente invention concerne un procédé de synthèse du perindopril, et de ses sels pharmaceutiquement acceptables, caractérisé en ce que l'on fait réagir le composé de formule (II) : 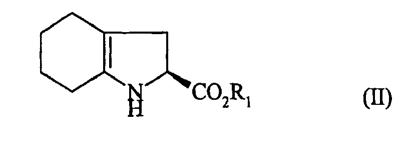 dans laquelle R₁ représente un atome d'hydrogène ou un groupement benzyle ou alkyle (C₁-C₆) linéaire ou ramifié,
avec un composé de formule (III), de configuration S : 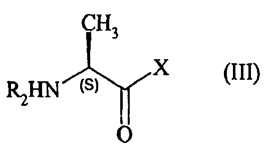 dans laquelle X représente un atome d'halogène, et R₂ représente un groupement protecteur de la fonction amino,
en présence d'une base,
pour conduire, après déprotection de la fonction amino, au composé de formule (IV) : 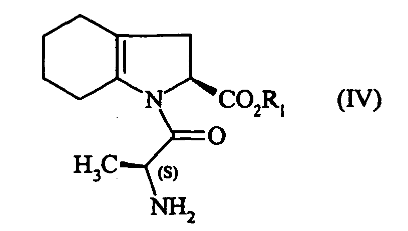 dans laquelle R₁ est tel que défini précédemment,
que l'on met en réaction avec le composé de formule (V) : 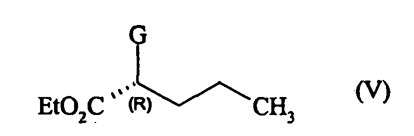 dans laquelle G représente un atome de chlore, de brome ou d'iode ou un groupement p-toluènesulfonyloxy, méthanesulfonyloxy ou trifluorométhanesulfonyloxy,
en présence d'une base,
pour conduire au composé de formule (VI) : 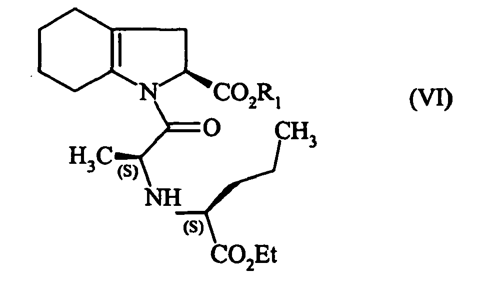 dans laquelle R₁ est tel que défini précédemment,
que l'on hydrogène en présence d'un catalyseur tel que le palladium, le platine, le rhodium ou le nickel,
pour conduire, après déprotection le cas échéant, au composé de formule (I).

Parmi les groupements protecteurs de la fonction amino utilisables dans le procédé de la présente invention, on peut citer à titre non limitatif les groupements tert-butyloxycarbonyle et benzyle.

R₁ représente préférentiellement le groupement benzyle. Dans ce cas, le groupement protecteur de la fonction amino est préférentiellement le groupement tert-butyloxycarbonyle.

Parmi les bases utilisables dans la réaction entre les composés de formules (II) et (III) ou entre les composés de formule (IV) et (V), on peut citer à titre non limitatif les amines organiques telles que la triéthylamine, la pyridine, la N-méthylmorpholine ou la diisopropyléthylamine, et les bases minérales telles que NaOH, KOH, Na₂CO₃, K₂CO₃, NaHCO₃ ou KHCO₃.

### EXEMPLE 1 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-1-{(2S)-2-[(Tert-butyloxycarbonyl)-amino]-propionyl}-2,3,4,5,6,7-hexahydro-1H-indole-2-carboxylate de benzyle

Dans un réacteur, charger 200 g de (2S)-2,3,4,5,6,7-hexahydro-lH-indole-2-carboxylate de benzyle, 1,5 1 de dichlorométhane, puis amener la température du mélange réactionnel à 0°C et ajouter 107 ml de triéthylamine, puis 162 g de chlorure de (2S)-2-[(tert-butyloxycarbonyl)-amino]-propionyle. Amener ensuite le mélange à température ambiante. Après 1h d'agitation à cette température, laver le mélange à l'eau puis avec une solution diluée d'acide acétique. La solution de (2S)-1-{(2S)-2-[(tert-butyloxycarbonyl)-amino]-propionyl}-2,3,4,5,6,7-hexahydro-1*H*-indole-2-carboxylate de benzyle ainsi obtenue est engagée telle quelle dans l'étape suivante.

### Stade B : (2S)-1-{(2S)-2-Aminopropionyl}-2,3,4,5,6,7-hexahydro-1H-indole-2-carboxylate de benzyle

Dans un réacteur, charger la solution obtenue au stade précédent, puis ajouter 133 g d'acide trifluoroacétique. Après 1h30 d'agitation à température ambiante, laver le mélange à l'eau, puis avec une solution saturée de bicarbonate de soude, et évaporer les solvants, pour conduire au (2S)-1-{(2S)-2-aminopropionyl}-2,3,4,5,6,7-hexahydro-1*H*-indole-2-carboxylate de benzyle.

### Stade C : (2S)-1-{(2S)-2-[(1S)-1-(Ethoxycarbonyl)-butylamino]-propionyl}-2, 3, 4, 5, 6, 7-hexahydro-1H-indole-2-carboxylate de benzyle

Dans un réacteur, charger 200 g du composé obtenu au stade précédent, 106 ml de diisopropyléthylamine et 1,5 1 de tétrahydrofurane, puis 183 g de (2R)-2-p-toluènesulfonyloxy-pentanoate d'éthyle, puis chauffer à 70°C pendant 2 h. Après retour à température ambiante, le milieu est lavé à l'eau puis mis à sec. Le résidu est repris dans le dichlorométhane. Une solution d'acide chlorhydrique (2M) est additionnée jusqu'à pH environ 7,5. Après décantation, les solvants sont évaporés pour conduire au (2S)-1-{(2S)-2-[(1S)-1-(éthoxycarbonyl)-butylamino]-propionyl}-2,3,4,5,6,7-hexahydro-1H-indole-2-carboxylate de benzyle.

### Stade D : Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 200 g du composé obtenu dans le stade précédent, en solution dans l'acide acétique, puis 5 g de Pt/C à 10 %. Hydrogéner sous pression de 5 bars entre 15 et 30°C, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant.
L'acide (2S, 3aS, 7aS)-1-{(2S)-2-[(1S)-1-(éthoxycarbonyl)-butylamino]-propionyl}-octahydro-1*H*-indole-2-carboxylique est ainsi obtenu avec un rendement de 85 % et une pureté énantiomérique de 98 %.

### Stade E : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le précipité obtenu dans le stade précédent (200 g) est mis en solution dans 2,8 1 d'acétonitrile, puis 40 g de tert-butylamine et 0,4 1 d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C. Le précipité obtenu est alors filtré, réempâté à l'acétonitrile, séché puis recristallisé dans l'acétate d'éthyle pour conduire au produit attendu avec un rendement de 95% et une pureté énantiomérique de 99%.

### EXEMPLE 2 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stades A et B : identiques aux stades A et B de l'exemple 1.

### Stade C : (2S)-1-{(2S)-2-[(1S)-1-(Ethoxycarbonyl)-butylamino]-propionyl}-2, 3, 4, 5, 6, 7-hexahydro-1H-indole-2-carboxylate de benzyle

Dans un réacteur, charger 200 g du composé obtenu au stade précédent, 106 ml de diisopropyléthylamine et 1,5 l d'acétate d'éthyle, puis 165 g de (2R)-2-chloro-pentanoate d'éthyle, puis chauffer à 50°C pendant 3h. Après retour à température ambiante, le milieu est lavé à l'eau puis mis à sec. Le résidu est repris dans le dichlorométhane. Une solution d'acide chlorhydrique (2M) est additionnée jusqu'à pH environ 7,5. Après décantation, les solvants sont évaporés pour conduire au (2S)-1-{(2S)-2-[(1S)-1-(éthoxycarbonyl)-butylamino]-propionyl}-2,3,4,5,6,7-hexahydro-1H-indole-2-carboxylate de benzyle.

*Stades D et E : identiques aux stades D et E de l'exemple 1.*

## Revendications

1. Procédé de synthèse des composés de formule (I) : 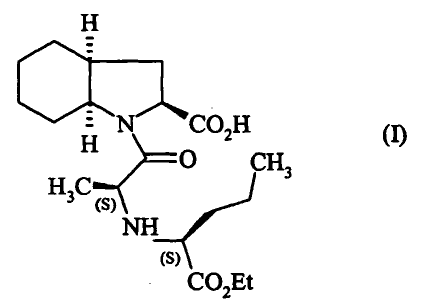 et de ses sels pharmaceutiquement acceptables,
**caractérisé en ce que** l'on fait réagir le composé de formule (II): 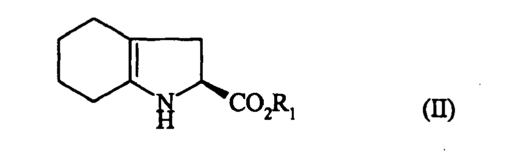 dans laquelle R₁ représente un atome d'hydrogène ou un groupement benzyle ou alkyle (C₁-C₆) linéaire ou ramifié,
avec un composé de formule (III), de configuration S : 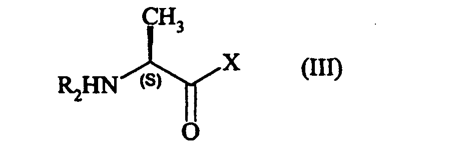 dans laquelle X représente un atome d'halogène, et R₂ représente un groupement protecteur de la fonction amino,
en présence d'une base,
pour conduire, après déprotection de la fonction amino, au composé de formule (IV) : 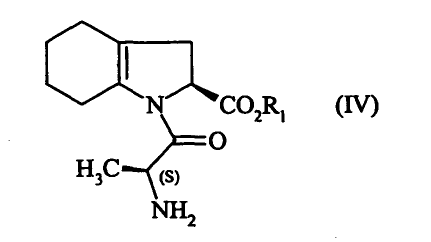 dans laquelle R₁ est tel que défini précédemment,
que l'on met en réaction avec le composé de formule (V) : 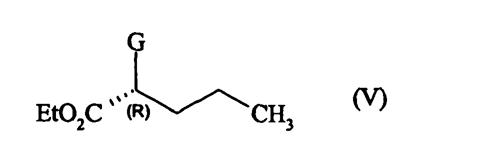 dans laquelle G représente un atome de chlore, de brome ou d'iode ou un groupement p-toluènesulfonyloxy, méthanesulfonyloxy ou trifluorométhanesulfonyloxy,
en présence d'une base,
pour conduire au composé de formule (VI) : 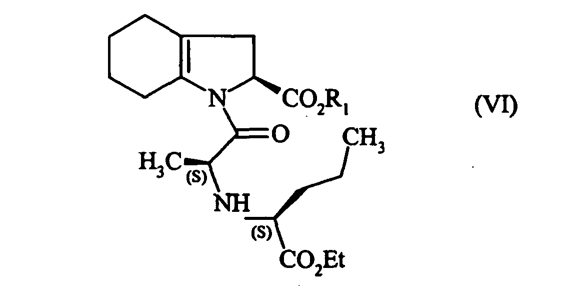 dans laquelle R₁ est tel que défini précédemment,
que l'on hydrogène en présence d'un catalyseur tel que le palladium, le platine, le rhodium ou le nickel,
pour conduire, après déprotection le cas échéant, au composé de formule (I).

2. Procédé de synthèse selon la revendication 1, **caractérisé en ce que** le groupement protecteur de la fonction amino est un groupement tert-butyloxycarbonyle ou benzyle.

3. Procédé de synthèse selon la revendication 2, **caractérisé en ce que** R₁ représente un groupement benzyle, et le groupement protecteur de la fonction amino est un groupement tert-butyloxycarbonyle.

4. Procédé de synthèse selon l'une quelconque des revendications 1 à 3, **caractérisé en ce que** la base utilisée pour la réaction entre les composés de formules (II) et (III) est une amine organique choisie parmi la triéthylamine, la pyridine, la N-méthylmorpholine et la diisopropyléthylamine, ou une base minérale telle que NaOH, KOH, Na₂CO₃, K₂CO₃, NaHCO₃ ou KHCO₃.

5. Procédé de synthèse selon l'une quelconque des revendications 1 à 4, **caractérisé en ce que** la base utilisée pour la réaction entre les composés de formules (IV) et (V) est une amine organique choisie parmi la triéthylamine, la pyridine, la N-méthylmorpholine et la diisopropyléthylamine, ou une base minérale telle que NaOH, KOH, Na₂CO₃, K₂CO₃, NaHCO₃ ou KHCO₃.

6. Procédé de synthèse selon l'une quelconque des revendications 1 à 5 du perindopril sous sa forme de sel de tert-butylamine.

## Claims

1. Process for the synthesis of the compound of formula (I) : 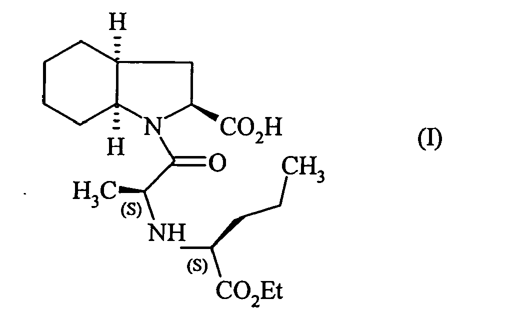 and its pharmaceutically acceptable salts,
**characterised in that** a compound of formula (II) : 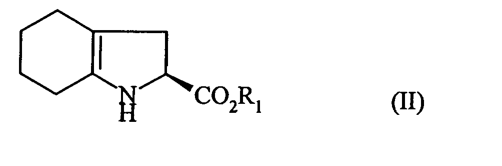 wherein R₁ represents a hydrogen atom or a benzyl or linear or branched (C₁-C₆)alkyl group,
is reacted with a compound of formula (III) having the S configuration : 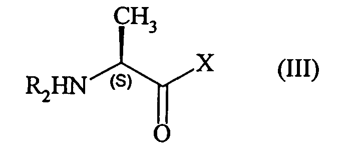 wherein X represents a halogen atom and R₂ represents a protecting group for the amino function,
in the presence of a base,
to yield, after deprotection of the amino function, a compound of formula (IV) : 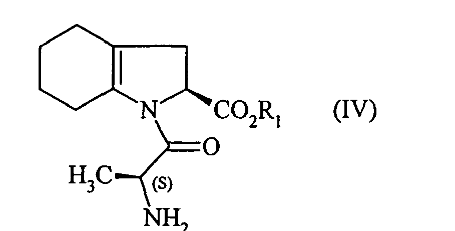 wherein R₁ is as defined hereinbefore,
which is reacted with a compound of formula (V) : 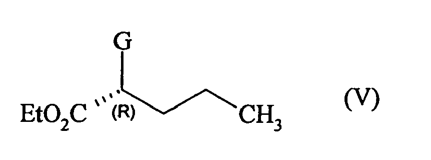 wherein G represents a chlorine, bromine or iodine atom or a p-toluenesulphonyloxy, methanesulphonyloxy or trifluoromethanesulphonyloxy group,
in the presence of a base,
to yield a compound of formula (VI) : 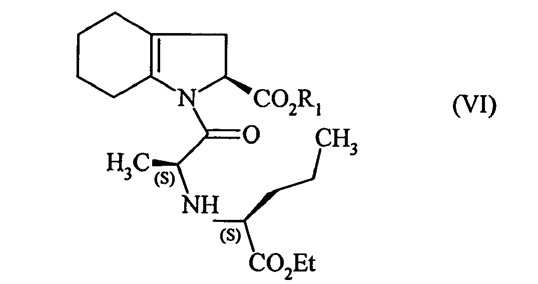 wherein R₁ is as defined hereinbefore,
which is hydrogenated in the presence of a catalyst such as palladium, platinum, rhodium or nickel
to yield, after deprotection where necessary, the compound of formula (I).

2. Synthesis process according to claim 1, **characterised in that** the protecting group for the amino function is a tert-butoxycarbonyl or benzyl group.

3. Synthesis process according to claim 2, **characterised in that** R₁ represents a benzyl group, and the protecting group for the amino function is a tert-butoxycarbonyl group.

4. Synthesis process according to any one of claims 1 to 3, **characterised in that** the base used for the reaction between the compounds of formulae (II) and (III) is an organic amine selected from triethylamine, pyridine, N-methylmorpholine and diisopropylethylamine, or a mineral base such as NaOH, KOH, Na₂CO₃, K₂CO₃, NaHCO₃ or KHCO₃.

5. Synthesis process according to any one of claims 1 to 4, **characterised in that** the base used for the reaction between the compounds of formulae (IV) and (V) is an organic amine selected from triethylamine, pyridine, N-methylmorpholine and diisopropylethylamine, or a mineral base such as NaOH, KOH, Na₂CO₃, K₂CO₃, NaHCO₃ or KHCO₃.

6. Process according to any one of claims 1 to 5 for the synthesis of perindopril in the form of its tert-butylamine salt.

## Patentansprüche

1. Verfahren zur Synthese von Verbindungen der Formel (I): 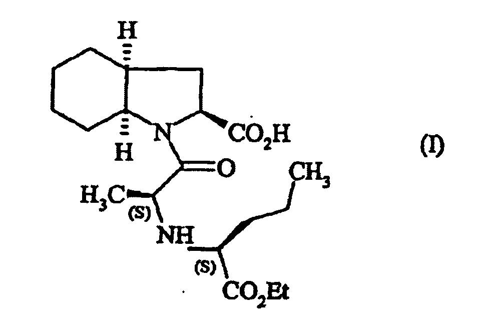 und von ihren pharmazeutisch annehmbaren Salzen,
**dadurch gekennzeichnet, daß** man die Verbindung der Formel (II): 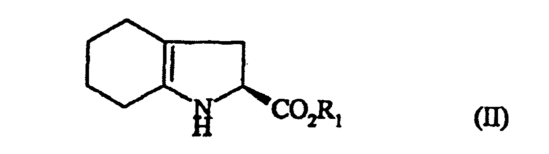 in der R₁ ein Wasserstoffatom oder eine Benzylgruppe oder geradkettige oder verzweigte (C₁₋C₆)-Alkylgruppe bedeutet,
mit einer Verbindung der Formel (III), der Konfiguration S: 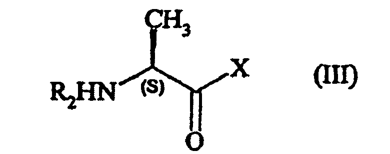 in der X ein Halogenatom und R₂ eine Schutzgruppe für die Aminofunktion bedeuten,
in Gegenwart einer Base umsetzt, so daß man nach der Abspaltung der Schutzgruppe der Aminofunktion die Verbindung der Formel (IV) erhält: 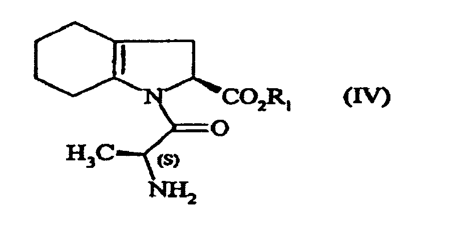 in der R₁ die oben angegebenen Bedeutungen besitzt,
welche man mit der Verbindung der Formel (V): 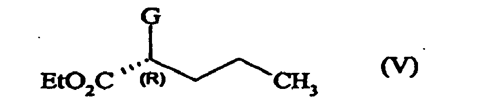 in der G ein Chloratom, Bromatom oder Iodatom oder eine p-Toluolsulfonyloxygruppe, Methansulfonyloxygruppe oder Trifluormethansulfonyloxygruppe bedeutet,
in Gegenwart einer Base umsetzt
zur Bildung der Verbindung der Formel (VI): 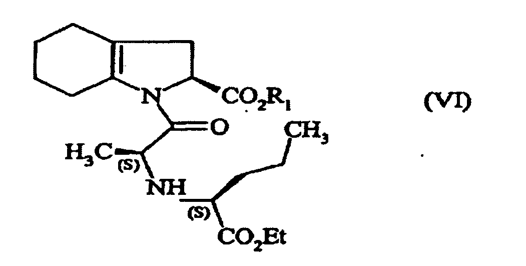 in der R₁ die oben angegebenen Bedeutungen besitzt,
welche man in Gegenwart eines Katalysators, wie Palladium, Platin, Rhodium oder Nickel, hydriert,
so daß man gegebenenfalls nach der Abspaltung der Schutzgruppe die Verbindung der Formel (I) erhält.

2. Syntheseverfahren nach Anspruch 1, **dadurch gekennzeichnet, daß** die Schutzgruppe der Aminofunktion eine tert.-Butyloxycarbonylgruppe oder Benzylgruppe ist.

3. Syntheseverfahren nach Anspruch 2, **dadurch gekennzeichnet, daß** R₁ eine Benzylgruppe bedeutet und die Schutzgruppe der Aminofunktion eine tert.-Butyloxycarbonylgruppe ist.

4. Syntheseverfahren nach einem der Ansprüche 1 bis 3, **dadurch gekennzeichnet, daß** die für die Reaktion der Verbindungen der Formeln (II) und (III) verwendete Base ein organisches Amin ausgewählt aus Triethylamin, Pyridin, N-Methylmorpholin und Diisopropylethylamin, oder eine anorganische Base, wie NaOH, KOH, Na₂CO₃, K₂CO₃, NaHCO₃ oder KHCO₃ ist.

5. Syntheseverfahren nach einem der Ansprüche 1 bis 4, **dadurch gekennzeichnet, daß** die für die Reaktion der Verbindungen der Formeln (IV) und (V) eingesetzte Base ein organisches Amin ausgewählt aus Triethylamin, Pyridin, N-Methylmorpholin und Diisopropylethylamin, oder eine anorganische Base, wie NaOH, KOH, Na₂CO₃, K₂CO₃, NaHCO₃ oder KHCO₃ ist.

6. Syntheseverfahren nach einem der Ansprüche 1 bis 5 für die Herstellung von Perindopril in Form seines tert.-Butylaminsalzes.
